Europäisches Patentamt

European Patent Office

Office européen des brevets

(19)

(11) **EP 0 992 250 A2**

(12) **EUROPEAN PATENT APPLICATION**

(43) Date of publication:
    **12.04.2000  Bulletin 2000/15**

(51) Int Cl.[7]: **A61L 15/60**

(21) Application number: **99307413.7**

(22) Date of filing: **20.09.1999**

(84) Designated Contracting States:
    **AT BE CH CY DE DK ES FI FR GB GR IE IT LI LU
    MC NL PT SE**
    Designated Extension States:
    **AL LT LV MK RO SI**

(30) Priority: **21.09.1998  US 157877
              07.09.1999  US 390460**

(71) Applicant: **FORT JAMES CORPORATION
    Deerfield, IL 60015 (US)**

(72) Inventor: **Bouchette, Michael Paul
    Sherwood, WI 54169 (US)**

(74) Representative: **Colmer, Stephen Gary et al
    Mathys & Squire
    100 Gray's Inn Road
    London WC1X 8AL (GB)**

(54) **Superabsorbent cellulosic natural or synthetic fibers and webs treated with polyelectrolytes and superabsorbent products made therefrom**

(57)    A superabsorbent fibrous web is disclosed. This web is formed from wood pulp or cellulosic fibers, natural fibers, synthetic fibers, or any mixture thereof wherein the web is treated with aqueous solutions of superabsorbent polyelectrolytes which produce a fibrous web having substantially increased absorbency rates and absorbent capacity. These webs are useful in the manufacture of towels, diapers, etc.

FIGURE 6

EP 0 992 250 A2

## Description

### RELATED APPLICATIONS

This is a continuation in part application of Serial No. 09/157,877 filed September 21, 1998.

### BACKGROUND OF THE INVENTION

This invention relates to novel superabsorbent cellulosic natural and synthetic webs and fibers containing polyelectrolytes that function as conventional chemical binders and/or adhesives and also exhibit superabsorbent properties. Although many of these chemical binders are called latex binders, in fact, they contain no natural latex and are instead synthetic organic polymers in solution and/or emulsions. The superabsorbent properties are valuable for paper products, textiles, and nonwovens, but the binding properties are critical for applications which in the prior art had to utilize separate absorbents and binders thus increasing the cost substantially.

### BACKGROUND

Prior art has been limited to superabsorbent powders or fibers. Powders not having the binding properties of the polyelectrolyte can not conveniently and economically be physically contained within an open and porous sheet structure. Superabsorbent fibers are very expensive and therefore can not be used in commercial nonwoven and absorbent paper manufacturing operations, which are very price sensitive.

Thus my invention meets an urgent need of the industry. Using my process the polyelectrolyte treated fibers and webs act as superabsorbents. Representative prior art includes the following United States Patents: Gross et al. 4,008,353; Oczkowski et al. 4,354,487; Roller 4,443,492; Dabi 4,645,789; Arroyo 4,867,526; Katz et al 4,888,238; Dabi et al 4,933,390; Dabi et al 4,944,963; Itoh et al 4,948,659; Manning et al 5,071,681; Hollenberg 5,126,382; Umeda et al 5,204,175; Arroyo et al 5,373,100; and Guersen et al 5,534,304.

None of the prior art references cited above suggest or disclose my novel superabsorbent web treated with polyelectrolytes such as the sodium, potassium or lithium salt of polyacrylic acid which in addition to exhibiting superabsorbent properties also function as a binder or adhesive and which thus enables the substrates to easily and economically retain superabsorbent properties without the difficulties of performance and/or cost exhibited in the prior art.

Prior art web forming technologies offer various ways of bonding the fibers together. Some of the various techniques may be classified as binderless, heat bonding, surface activation bonding, and chemical bonding. Binderless bonding is achieved through physical entanglement of the fibers. Heat bonding is bonding achieved through melting of certain fibers or fiber surfaces to each other or other fibers. Surface activation bonding is done through a chemical treatment which renders the fibers of the surface partially softened and reactive so that they can flow together, and when they re-solidify, bonds are formed. Chemical bonding uses a chemical itself. At points where the fibers are touching each other, chemical encapsulation and/or chemical-to-fiber crosslinking bonds the fibers together. My invention applies to all nonwoven, paper, and textile webs or substrates. The greatest benefit for my invention is found in those substrates that require chemical bonding, because my invention uses the superabsorbent polyelectrolyte in combination with a crosslinker as the binder.

With a focus on the nonwovens industry, the substrate is first formed from any combination of cellulose or wood pulp, natural, and/or synthetic fibers. These webs need to be bonded because they have little to no strength or integrity under stress. Thus the chemical binder has to be sprayed, printed, or otherwise applied to the web to give it integrity and utility. For example, in air laid nonwovens, the substrate is comprised of wood pulp, or wood pulp and synthetic fibers. (Superabsorbent fibers are included in the definition of synthetic fibers.) While the substrate formed has very little (but nonetheless some) dry integrity, the process of final substrate preparation generally requires at least some compaction or embossing to densify the substrate and increase entanglement for in-process handling strength. Typically this compaction increases physical entanglement and touching of the fibers, and also generates a small amount of hydrogen bonding at fiber touch points. But generally this step is still insufficient to add significant strength to render the final product useful. Tensile strengths of the unbonded web both before and after typical process compactions are extremely low, if measurable at all. For example, tensile strengths of an unbonded web that was sprayed with water and then dried to enhance hydrogen bonding still further is only about 200 gr/in. This is still insufficient for most applications, particularly those that have to undergo the stress of various converting operations. Therefor, a chemical binder is added to the web as one way for ultimate strength development. My invention eliminates or greatly reduces the need for conventional binders while producing nonwovens, paper, or textile products having superior absorbent properties.

Using air laid web formulation as an example, a newer technology for bonding is the incorporation of bicomponent synthetic fibers in the web furnish. These fibers are such that when heated the sheath of the fiber melts. Wherever these synthetic fibers touch any other fiber (wood pulp included), they will bond when the melted sheath is cooled. But these bicomponent-bonded webs, also known as thermal bonded webs to those in the industry, have also shown some major deficiencies. Namely, they are not as strong as chemically bonded webs, and the overall bonding sites are apparently less secure as demonstrated by the fact that webs of this nature tend to lint a lot. A "fix" for this problem has been to spray the surface of these webs lightly with a chemical binder to tie down loose surface fibers. Thus the need for chemical binders still exists in many of these types of webs. In my invention the use of a conventional chemical binder is eliminated or greatly reduced, and it is replaced with my superabsorbent polyelectrolyte which not only binds the loose fibers on the surface and reduces linting, but also has superabsorbent properties. In addition my superabsorbent polyelectrolyte treated products have superior absorbent properties when compared to conventional air laid nonwoven products utilizing latex binders. Thus my invention is greatly superior to the prior art.

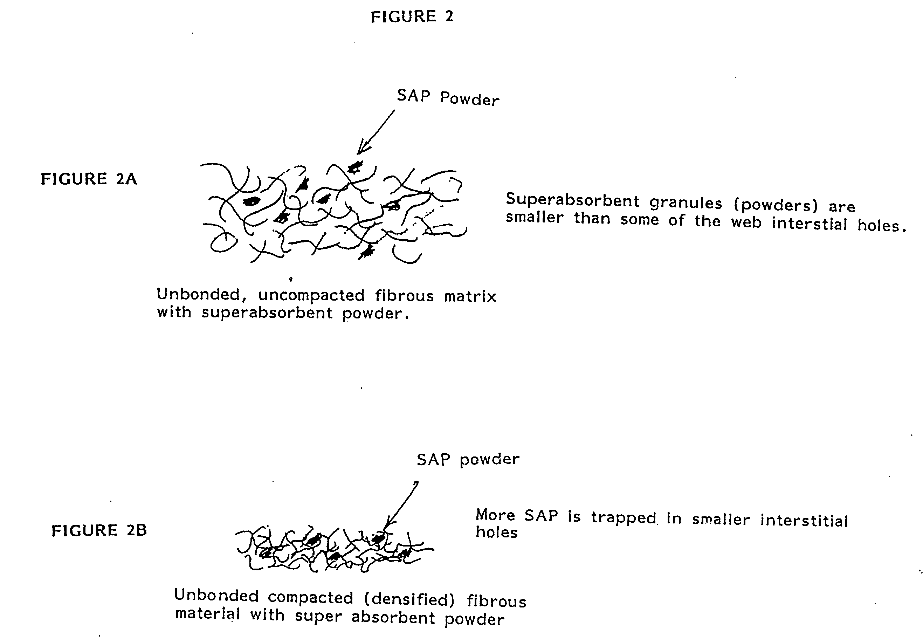

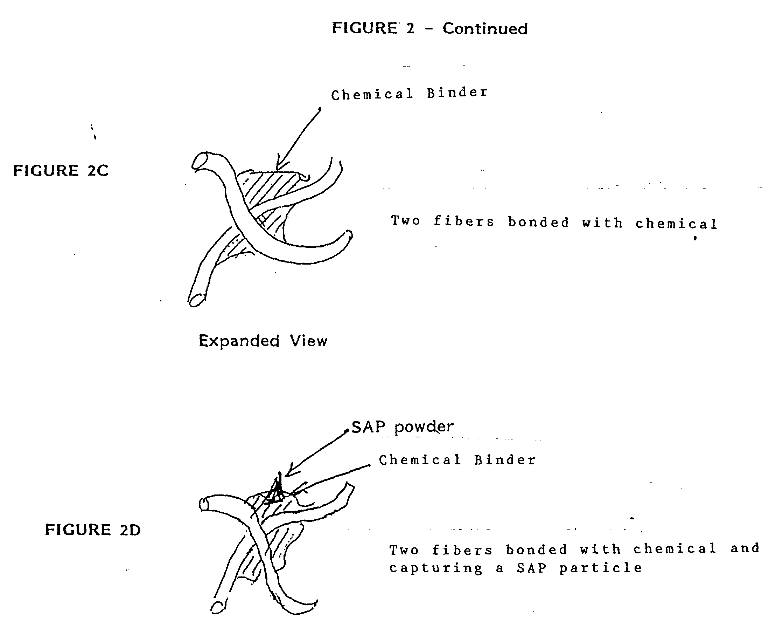

In order to achieve truly superabsorbent properties in various web substrates, it is necessary to add a superabsorbent chemical. Superabsorbents today are commercially found in the forms of powders or fibers. Powders have the advantage of relatively low cost, but the disadvantage of being so small in particle size as to fall through the holes or interstices of the substrate as shown in Figure 2A. Therefore, the user does not realize the full economic benefit of the powder because of the loss of the powder through handling and converting. Enhancing the particle size of powders has been tried in the prior art but is an unsatisfactory solution since the surface area of the superabsorbent is reduced and thereby causes gel blocking when wetted. This reduces the efficiency of this approach. The aforementioned process also suffers from the fact that it requires that large particles be placed in small holes, thus making the process uneconomical.

One way to increase the retention of superabsorbents (SAP) is to densify the web making the interstitial voids much smaller so the SAP cannot fall out as easily. This is shown in Figure 2B. However, web densification is not always desirable or acceptable where a bulky product is preferred. Furthermore, densification reduces the ability of the SAP to swell and expand when wetted, thereby negating some of its value. Neither an increase in particle size nor a decrease in interstitial hole size relieves the deficiencies of unbonded SAP on the surface of the webs where it can still fall off. Another way to increase retention of the SAP is to either top coat or saturate the web with a conventional chemical binder. As the binder dries, it retreats in its volume of coverage to minimize surface tension. Typically it gathers at fiber crossover points or where an SAP particle is resting on a fiber. In this action, it then bonds fibers together and fibers and SAP together. This is shown in Figures 2C and 2D. Chemical bonding the SAP to or within the substrate is an effective way of retaining the SAP, but it also increases the risk of encapsulating the SAP thereby reducing or eliminating its ability to be exposed to water or the insult liquid and thereby reducing its effectiveness.

Fibers are far more expensive than powders and do not have the deficiencies of the powders, but most web-forming systems have difficulty in handling synthetic and/or superabsorbent fibers. Both the handling deficiencies and the economic deficiencies of prior art superabsorbents are overcome by my invention through the use of a polyelectrolyte functioning as a binder having superabsorbent properties. The superabsorbent fibers may be retained in the web through binderless bonding (mechanical entanglement), heat bonding, surface activation bonding, chemical bonding, and the like. In Figure 2C, showing a chemical bond between two fibers, one or more of these fibers may be superabsorbent fibers (SAF). In my invention the chemical bond itself is superabsorbent negating the need for the expensive and difficult to handle superabsorbent fiber.

It is often desirable to incorporate the addition of superabsorbents in other nonwoven web forming and web bonding technologies, which do not normally require chemical bonding. Most often, the addition of superabsorbents to such substrates is still frustrated by the handling and inadequate retention of powders, or by the cost and handling of fibers. My invention overcomes these disadvantages of the prior art since in my invention, the polyelectrolyte used serves with dual functionality - as that of an adhesive or binder and as a superabsorbent.

Papermaking is another method of web forming technology. The addition of superabsorbents to the furnish, whether by powder or fiber generally cause too many other problems in the papermaking operation, namely the gelling of the furnish and increased drying loads. My invention overcomes these prior art disadvantages. The superabsorbent polyelectrolyte solution polymer in combination with a crosslinker is formulated as a low viscosity water solution polymer, and can be added right into the papermaking furnish without the handling problems encountered in the prior art.

Textiles are another form of web making technology. Textiles are usually made by weaving or knitting. Where it is unusual that textiles require latex bonding for strength enhancement, superabsorbent properties may still be desirable. The addition of superabsorbent powders cannot be done easily at all in knitting or weaving operations. It is more difficult to incorporate superabsorbent fibers into the yarns used in knitting or weaving. In my invention the superabsorbent binder can be applied to the textile web where it is bonded to the web by its binder properties and whereby it still exhibits superabsorbent properties.

My invention can also be used in papermaking, nonwovens, and textiles as either a surface or laminating adhesive and/or textiles, thereby delivering superabsorbent properties to virtually any web-like final product or product construction which requires superabsorbents to function in the absorption of liquids.

## SUMMARY OF THE INVENTION

This invention relates to a super absorbent fibrous web formed from cellulosic fibers, natural fibers, synthetic fibers, or any combination thereof wherein the web is treated with aqueous solutions of superabsorbent polyelectrolytes which produce a fibrous web having substantially increased absorbency rates and absorbent capacity. In addition the invention relates to a superabsorbent, nonwoven, fibrous web formed from cellulosic fibers, a mixture of wood pulp and synthetic fibers, or 100 percent synthetic fibers, wherein aqueous solutions of superabsorbent polyelectrolytes replace conventional binders and which produce superabsorbent nonwovens having substantially increased absorbency rates and absorbent capacity. The treated web of my invention uses a polyelectrolyte solution polymer with the combined properties of superior absorbency and bonding. The absorbent polyelectrolyte in combination with the crosslinker functions to hold the fiber together and to impart in the web superabsorbent properties. In addition the superabsorbent polyelectrolyte and crosslinker can be applied as a topcoat to the web to enhance superabsorbent properties. In certain applications the polyelectrolyte and crosslinker is applied as a laminate adhesive to hold two or more webs together while also enhancing superabsorbent properties.

The polyelectrolyte is selected from the group consisting of polyacrylate polymers and their sodium, lithium or potassium salts, wherein the polyacrylate has a molecular weight in excess of 150,000. Suitably a molecular weight of about 150,000 to 220,000, preferably a molecular weight of about 190,000 to 220,000.

The polyelectrolyte in the presence of a crosslinker can suitably be sprayed, padded, printed, saturated, or otherwise so coated applied to the substrate. Suitable products made utilizing the superabsorbent polyelectrolyte include towels, feminine hygiene pads, incontinence pads, baby and adult diapers, wound dressing, medical drapes, meat soaker pads, wipers, cable wrap papers and agricultural moisture retention systems.

The nonwoven web is formed by one of the following processes: (1) air laid, (2) carding, (3) melt blown, (4) spunbond, and (5) coforming. The textile web is formed by either a weaving or knitting process. The paper web is formed by fourdrinier, roof formers, gap formers, cylinder forming, or rotoforming paper making processes.

## BRIEF DESCRIPTION OF THE DRAWINGS

Figure 1A illustrates a typical unbonded fibrous matrix. This matrix may be all wood pulp or cellulosic fibers, all natural fibers, all synthetic fibers, or any mixture thereof. Figure 1A shows discontinuous fibers as would be found in nonwovens and paper. In the case of textiles, the fibrous matrix would be composed of woven or knitted yarns. The yarns themselves would be comprised of intertwined discontinuous fibers, continuous fibers, or blends thereof.

Figure 1B illustrates an expanded view of a superabsorbent fiber contrasted with a non-superabsorbent fiber.

Figure 2A illustrates a typical unbonded discontinuous fibrous matrix with superabsorbent granules (powder) in the web interstices. The size of the granules with relationship to the interstitial void size allows some particles to filter through and fall out. The fibers themselves could be wood pulp or cellulosic fibers, natural fibers, synthetic fibers, or any mixture thereof.

Figure 2B illustrates that retention of the superabsorbent powder granules can be enhanced through web densification, but surface granules can still fall off.

Figure 2C illustrates a typical chemical bonded discontinuous fibrous matrix showing the chemical bonding the fibers together. The fibers may be wood pulp, natural, synthetic, or mixtures thereof. The chemical may bond surface to surface (as shown) or may actually encapsulate the fibers. The chemical binder may be a superabsorbent binder (SAB) or conventional.

Figure 2D shows two fibers bonded with a chemical while also capturing a SAP particle. The fibers may be wood pulp or cellulosic fibers, natural fibers, synthetic fibers, or combinations thereof. The chemical binders may be surface to surface as shown or may encapsulate some of the fiber. The chemical may be SAB or conventional.

Figure 3A illustrates an expanded view of a typical thermal bonded fibrous matrix comprised of wood pulp or cellulosic fibers, natural fibers, synthetic fibers, and bicomponent fibers in any mixture or bicomponent fibers alone whereby the bicomponent fibers are bonded at the fiber crossover points.

Figure 3B illustrates a fiber bonded crossover point using bicomponent fibers for bonding wherein the melted fiber sheath has also captured an SAP particle.

Figure 4A illustrates typical unbonded fibrous matrix.

Figure 4B illustrates fiber crossover point of unbonded fibers.

Figure 5A illustrates a typical cross section of fibrous matrix wherein the fibers may be cellulosic, natural, synthetic, or combinations thereof.

Figure 5B illustrates the densified matrix of Figure 5A

Figure 6 is a schematic drawing of the process for the manufacture of a super absorbent, air laid, nonwoven product of this invention.

Figure 7 is a schematic flow diagram of a papermaking process showing suitable points of addition of the aqueous superabsorbent polyelectrolyte.

Figure 8 illustrates a through air drying (TAD) process for the manufacture of the' superabsorbent paper products of this invention. In the TAD process the aqueous superabsorbent polyelectrolyte can be printed after the web has been dried or alternatively the aqueous superabsorbent polyelectrolyte can be sprayed on the dried TAD web or prior to drying of the web.

Figures 9A and 9B illustrate suitable direct gravure printing processes. In Figure 9B, 102A is the fountain pan, and 102B is the oscillating doctor blade.

Figures 10A and Figure 10B illustrate suitable flexographic printing processes. In Figure 10A, 105 is impression roll; 106 is plate roll; 108 is engraved anilox roll; 109 is aqueous superabsorbent polyelectrolyte supply; and 113 is manifold. In Figure 10B, 111 is a rubber fountain roller, and 112 is a fountain pan.

Figures 11A and Figure 11B illustrate suitable offset gravure processes.

Figures 12A, 12B, and 12C illustrate suitable press designs, a central impression, stack and in-line flexographic press design.

Figure 13 is a schematic drawing of the process for utilizing a carding process for the manufacture of nonwovens.

## DETAILED DESCRIPTION OF THE INVENTION

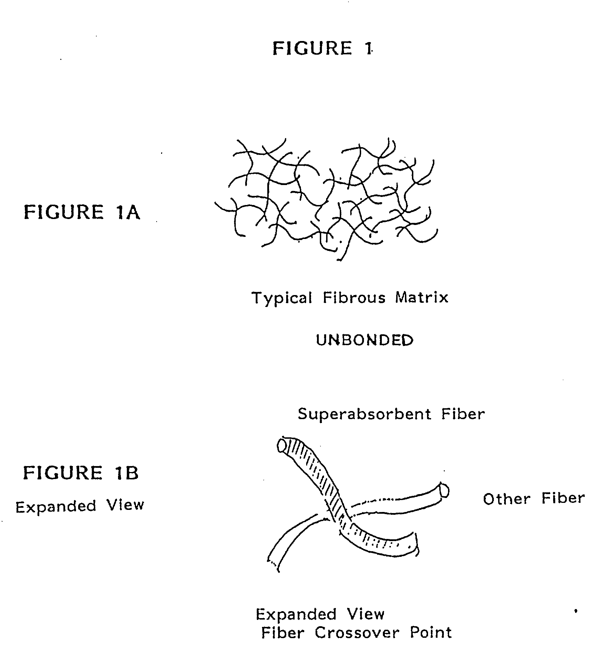

A superabsorbent fibrous web formed from wood pulp or cellulosic fibers, natural fibers, synthetic fibers, or any mixture thereof wherein the web is treated with aqueous solutions of superabsorbent polyelectrolytes in combination with crosslinkers which produce a fibrous web having substantially increased absorbency rates and absorbent capacity are disclosed. The superabsorbent webs are illustrated in Figures 2C, and 2D where the chemical binder used is that superabsorbent polyelectrolyte and crosslinker used in my invention. Prior art webs and fibers are shown in Figures 1A, 1B, 2A, 2B, 2D, 3A, 3B, 4A, 4B, 5A and 5B.

The web may suitably be treated with the aqueous solution of superabsorbent polyelectrolyte and crosslinker wherein the treatment method may be printing, padding, spraying, saturation, etc. The polyelectrolyte binder system produces nonwovens, absorbent papers and textiles having substantially increased absorbency rates. The absorbency rates are increased by 10 to 50 or more percent and absorbency capacity is increased by 10 to 50 or more percent.

The superabsorbent polyelectrolyte is selected from polyacrylate polymers and their sodium, lithium, or potassium salts. A suitable polyelectrolyte has a molecular weight of at least 150,000 advantageously 190,000 to 220,000. Advantageously the superabsorbent web is converted to an absorbent product. The preferred absorbent paper product is a towel or wiper. The nonwoven and textile fibrous webs are advantageously converted to the following: (a) feminine hygiene pads, (b) incontinence pads, (c) adult diapers, (d) baby diapers, (e) wound dressings, (f) meat soaker pads, (g) wipers, (h) cable wrap papers, (i) agricultural moisture retention systems, and (j) medical drapes.

The alkali-soluble polyacrylates known as polyelectrolytes useful to form the crosslinkable solutions utilized in carrying out this invention can be made by utilizing processes such as emulsion, suspension, bulk or solution polymerization techniques, provided they consist of about 50 to 92 percent by weight of an alkyl acrylate with 1-10 carbon atoms in the alkyl moiety or an alkyl methacrylate with 4-10 carbon atoms in the alkyl moiety, about 8 to about 50 percent by weight of an olefinically unsaturated carboxylic acid and about 0 to about 15 percent by weight of an omega hydroxy alkyl acrylate having 1-4 carbons in the alkyl moiety.

It is preferred to use alkali-soluble latices having about 15 to about 60 weight percent of non-volatile polymer solids as is set forth below.

Examples of useful alkyl acrylates are methyl acrylate, ethyl acrylate, propyl acrylate, hexyl acrylate and the like. Examples of useful alkyl methacrylates are butyl methacrylates, hexyl methacrylates, octyl methacrylate, decyl methacrylate and the like

Examples of useful omega hydroxy alkyl acrylates are 2-hydroxyethyl acrylate, hydroxymethyl acrylate, 3-hydroxy-propyl acrylate and 4-hydroxybutyl acrylate.

The foregoing polyacrylates are then dissolved in an aqueous alkali metal hydroxide solution. Generally the equivalents of hydroxide solution used are from about 30 to about 70% based on the molar concentration of polymerized monomer and the preferred amount is from about 40 to about 55%. In any event, the amount of hydroxide solution added is sufficient to convert or saponify some of the acrylate esters to alkali metal carboxylates and to neutralize the carboxylic groups of the polyacrylate used into alkali metal carboxylates so that the converted polyacrylate has about 30 to about 70 weight percent of alkali metal carboxylates.

This solution is then rendered crosslinkable by adding about 0.1 to about 10 weight percent based on the dissolved polymer of a water soluble crosslinking agent.

Suitable crosslinking agents are zirconium compounds having a valence of plus four. Suitable zirconium compounds include ammonium zirconium carbonate, zirconium acetylacetonate, zirconium acetate, zirconium carbonate, zirconium sulfate, zirconium phosphate, potassium zirconium carbonate, zirconium sodium phosphate and sodium zirconium tartrate.

Other suitable soluble crosslinking agents are polyhaloalkanols such as 1,3-dichloroisopropanol, 1,3-dibromoisopropanol; sulfonium zwitterions such as the tetrahydrothiophene adduct of novolac resins; haloepoxyalkanes such as epichlorohydrin, epibromohydrin, 2-methyl epichlorohydrin and epidohydrin; polyglycidyl ethers such as glycerine diglycidyl ether, ethylene glycol diglycidyl ether, propylene glycol diglycidyl ether, diethylene glycol diglycidyl ether; and the mixtures of the foregoing.

The cellulosic fibers can suitably be substituted with synthetic fibers and/or natural fibers. The substitution can range from 10 to 50 percent. In some instances the cellulosic fiber is completely replaced with the synthetic fiber and/or natural fibers. Suitably synthetic fibers are selected from regenerated cellulose, polyolefins, polyethylenes, polypropylenes, polyesters, polyamines, polyamides, inorganic polymers, silicon polymers, and carbon fibers. Suitable natural fibers are selected from wool, camel hair, mohair, and other non-cellulosic animal, vegetable, or mineral sources.

Papermaking fibers used to form the paper products of the present invention include cellulosic fibers commonly referred to as wood pulp fibers, liberated in the pulping process from softwood (Gymnosperms or coniferous trees) and hardwoods (angiosperms or deciduous trees). Cellulosic fibers from diverse material origins may be used to form the web of the present invention including non-woody fibers liberated from sugar cane, bagasse, sabai grass, rice straw, banana leaves, paper mulberry (i.e., bast fiber), abaca leaves, pineapple leaves, esparto grass leaves, and fibers from the genus Hesperaloe in the family Agavaceae. Also recycled fibers which may contain any of the above fiber sources in different percentages can be used in the present invention. Suitable fibers are disclosed in U.S. Patent Nos. 5,320,710 and 3,620,911, both of which are incorporated herein by reference.

Papermaking fibers can be liberated from their source material by any one of the number of chemical pulping processes familiar to one experienced in the art including sulfate, sulfite, polysulfite, soda pulping, etc. The pulp can be bleached if desired by chemical means including the use of chlorine, chlorine dioxide, oxygen, etc. Furthermore, papermaking fibers can be liberated from source material by any one of a number of mechanical/chemical pulping processes familiar to anyone experienced in the art including mechanical pulping, thermomechanical pulping, and chemi thermomechanical pulping. These mechanical pulps can be bleached, if one wishes, by a number of familiar bleaching schemes including alkaline peroxide and ozone bleaching. The type of furnish is less critical than is the case for prior art products. In our process coarse hardwoods and softwoods and significant amounts of recycled fiber can be utilized to create useful products.

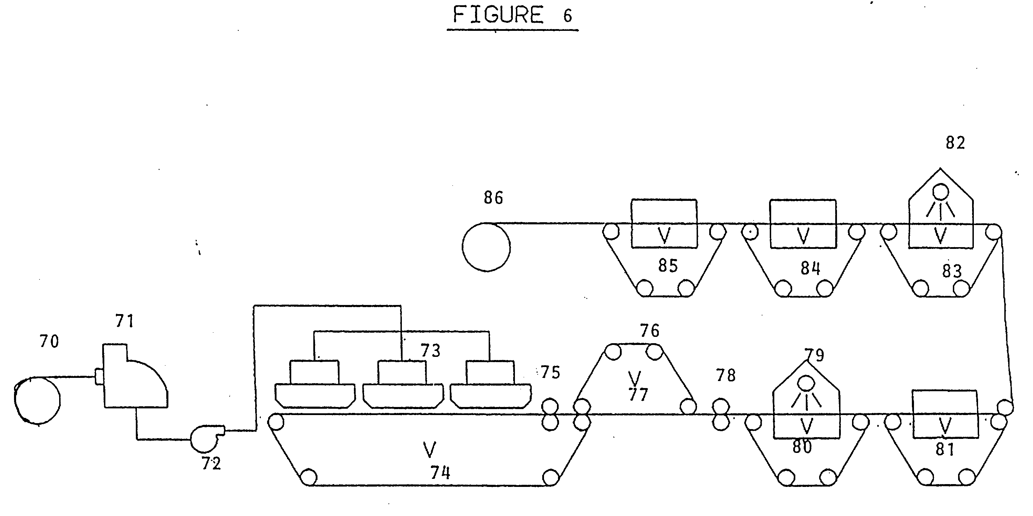

Figure 6 illustrates a preferred process for the manufacture of a super absorbent, air laid, nonwoven fibrous web of this invention.

Roll pulp (70) can be regular or debonded or it can be baled pulp when used with proper hammermill design. The roll pulp (70) is charged into hammermill defibrator (71). Suitably one or more, advantageously three to ten or more hammermills (71) are employed. In the hammermills also synthetic fibers may be introduced. In hammermill feeding, usually no more than 50 percent of the fibrous cellulose is replaced with synthetic fibers, natural fibers, or blends thereof. For some applications 100 percent synthetic fibers or natural fibers may be desirable. Synthetic fibers or natural fibers may also be introduced through other conventional long fiber opening equipment such as a LaRoche opener and feeder and fed directly into the air stream entering the forming head. Under these conditions, 100 percent synthetic fibers or natural fibers may be fed into the system. The synthetic fibers are selected from regenerated cellulose including rayon, polyolefins including polyethylenes and polypropylenes and their copolymers, polyesters, polyimides, polyamides, inorganic polymers, silica polymers and carbon fibers. Synthetic and/or natural fibers opened through a LaRoche or similar device are most typically added to the system through the air transport fan (72).

Air transport fiber (72) is used to carry the air and fiber mixture to multiple forming heads (73). In Figure 6 three forming heads (73) are shown. However, one, two, three, up to six or ten forming heads (73) can be used. Vacuum under foraminous wire (74) serves to aid in the fiber formation. Compaction rolls (75) are used to increase web integrity. These rolls (75) function to densify the web and aid in fiber entanglement and in case of cellulosic fibers increase hydrogen bonding. The strengthened web passes through the transfer section (76) to bonding. Emboss rolls (78) are used to impart aesthetic attributes and increase absorbency of final webs. Emboss rolls may also be located after the final drying oven (84) or curing oven (85).

In the first spray cabin (79), the aqueous superabsorbent polyelectrolyte including the crosslinker is sprayed on the web. Vacuum assist (80) of the aqueous superabsorbent polyelectrolyte binder spray takes place at this site. The first vacuum oven (81) is operated at a temperature of about 200°F to 500°F with vacuum assist. In the second polyelectrolyte binder spray cabin (82), the web is again sprayed with the superabsorbent polyelectrolyte binder and the crosslinker. The second drying takes place at oven (84) with vacuum assist. At site 85, further curing of the polyelectrolyte binder takes place. It is believed that most of the curing has taken place prior to site 85. The web is rolled on roller (86).

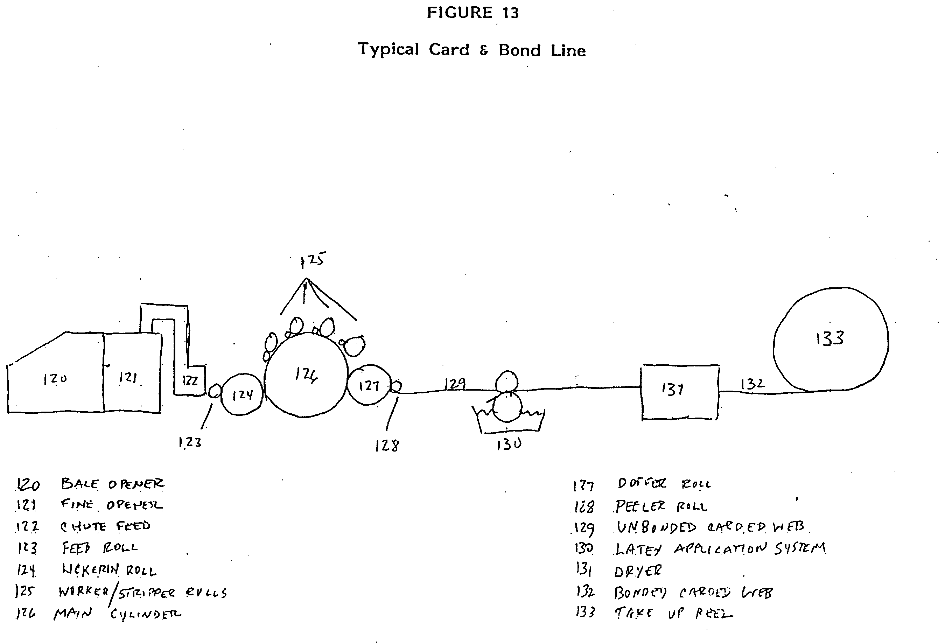

In a typical carding operation as shown in Figure 13, the bales of compacted fibers are first "opened" in the bale opener (120). This is a coarse action which decompacts the bale of fibers and, at least partially, separates the fibers from each other. Depending upon any number of factors including the type of fiber used, the opening may result in individualization of fibers, or it may result only in the formation of small clumps of fibers. From the bale opener (120), the fibers are transported to the fine opener (121). Here the opening process is continued and the fibers are further separated and declumped until they are ready for processing and forming. The fibers are then transported into the chute feed (122) which meters them at a uniform rate and volume to the feed roll (123) and then the lickerin roll (124) to transfer the fibers in a separated, uniform, and controlled manner. The fibers then are transferred from the lickerin roll (124) to the main cylinder (126). Here they are combed and stripped from the main cylinder (126) by the worker and stripper rolls (125). The web is then transferred to the doffer roll (127). From the doffer roll (127), the web is peeled off by the peeler roll (128) and is now ready for further processing. Further processing may be randomizing, which is not shown in this schematic. Randomizing is a method to increase alignment of the fibers in the CD direction to give increased homogeneity to the web with respect to fiber alignment. Other techniques such as cross-lapping may also be used if so desired. Regardless of the use of randomizing or other subsequent processing variables, in my invention the web must be bonded or coated with the superabsorbent polyelectrolyte binder in order to achieve the superabsorbent properties. If the web is truly bonded, as is indicated in Figure 13, the web is passed through a spray, printing, or padding application which applies sufficient superabsorbent polyelectrolyte binder to bond both the surface and interstitial fibers. In Figure 13, this is shown by the Application System (130). In mere coating, where the product objectives might be different in that ultimate strength development is not necessary, the superabsorbent binder used in my invention might only be coated on the surface or diluted sufficiently so as to still penetrate the web but not be present in sufficient quantity to add significant strength. In order to achieve only a coating effect in which the only strength requirements would be to hold the superabsorbent itself to the web or web surface, the application method and equipment might vary from saturation, spraying, or padding. However, regardless of the application method, the basic process chronology step of polyelectrolyte chemical binder application would still be done following complete web formation at a typical application system (130) point within the process. Following the polyelectrolyte chemical binder application, the wetted web would be dried in some type of a dryer (131). This may be steam cans, radiant heat, through air drying, or the like. At this point, the final bonded web (132) would then be wound up onto the reel (133) into a parent roll.

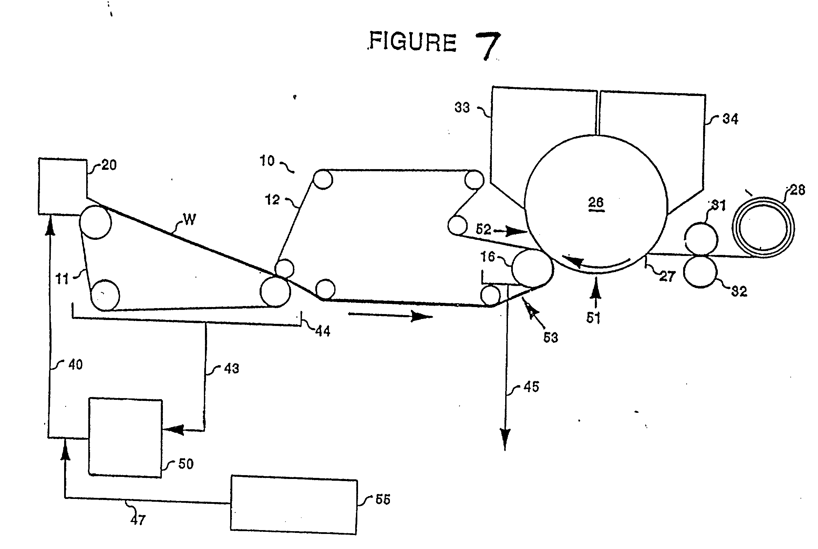

Figure 7 illustrates an embodiment of the present invention wherein machine chest (55) is used for preparing the papermaking furnish.

Superabsorbent polyelectrolytes including crosslinkers are added to the furnish in the machine chest (55) or in conduit (47). Optionally, dry strength agents and temporary or permanent wet strength agents may also be added. The furnish may be treated sequentially with chemicals having a different functionality depending on the character of the fibers that constitute the furnish, particularly their fiber length and coarseness, and depending on the precise balance of properties desired in the final product. The furnish is diluted to a low consistency, typically 0.5 percent or less, and transported through conduit (40) to headbox (20) of a paper machine (10). Figure 7 includes a web-forming end with a liquid permeable foraminous forming fabric (11) that may be of any conventional configuration.

A wet nascent web (W) is formed in the process by ejecting the dilute furnish from headbox (20) onto the forming fabric (11). The web is dewatered by drainage through the forming fabric and additionally by such devices as drainage foils and vacuum devices (not shown). The water that drains through the forming fabric may be collected in the wire pit (44) and returned to the papermaking process through conduit (43) to silo (50), from wherein it again mixes with the furnish coming from machine chest (55).

From the forming fabric (11), the wet web is transferred to felt (12). Additional dewatering of the wet web may be provided prior to thermal drying, typically by employing a non-thermal dewatering means. This non-thermal dewatering is usually accomplished by various means for imparting mechanical compaction to the web, such as vacuum boxes, slot boxes, contacting press rolls, or combinations thereof. The wet nascent web (W) is transferred to the drum of a Yankee dryer (26). Fluid is pressed from the wet web (W) by pressing roll (16) as the web is transferred to the drum of the Yankee dryer (26) at a fiber consistency of at least about 5% up to about 50%, preferably at least 15% up to about 45%, and more preferably to a fiber consistency of approximately 40%. The web is then dried by contact with the heated Yankee dryer and by impingement of hot air onto the sheet, said hot air being supplied by hoods (33) and (34). The web is then creped from the dryer by means of a creping blade (27). The finished web -may be pressed between calender rolls (31) and (32) and is then collected on a take-up roll (28).

Adhesion of the partially dewatered web to the Yankee dryer surface is facilitated by the mechanical compressive action exerted thereon, generally using one or more pressing rolls (16) that form a nip in combination with thermal drying means (26). This brings the web into more uniform contact with the thermal drying surface. The attachment of the web to the Yankee dryer may be assisted and the degree of adhesion between the web and the dryer controlled by application of various creping aids that either promote or inhibit adhesion between the web and the dryer (26). These creping aids are usually applied to the surface of the dryer (26) at position (51) prior to its contacting the web.

Also shown in Figure 7 are the locations for applying polyelectrolytes to the already formed cellulosic web. According to the preferred embodiment of the process of the invention, the superabsorbent polyelectrolyte including the crosslinker can be applied from position (52) or (53) on the airside of the web or on the Yankee side of the web respectively.

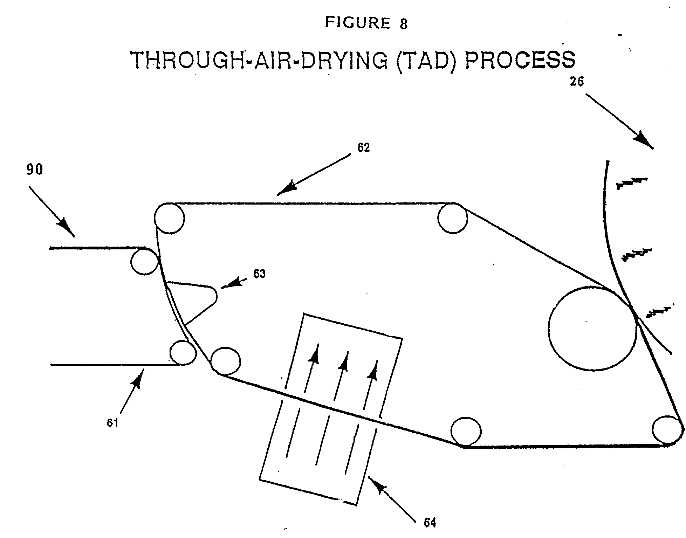

The through air drying (TAD) process is illustrated in Figure 8. In the process, wet sheet 90 that has been formed on forming fabric (61) is transferred to through air drying fabric (62), usually by means of vacuum device (63). TAD fabric (62) is usually a coarsely woven fabric that allows relatively free passage of air through both fabric (62) and nascent web (90). While on the fabric (62), the sheet (90) is dried by blowing hot air through the sheet (90) using through air drier (64). This operation reduces the sheet's moisture to a value usually between 20 and 95 percent. The partially dried sheet (90) is then transferred to Yankee dryer (26) where it is dried to its final desired moisture content and is subsequently creped off the Yankee. Alternatively, as shown in U.S. Patents 5,607,551; 5,048,589 and European Patent Applications EP0677612A3 and EP0617164A1, the drying can be conducted without the use of a Yankee dryer and creping. In our process any air drying means practiced in the art is suitable. All four of these references are incorporated herein by reference. The polyelectrolytes including the crosslinker are sprayed from an aqueous solution or printed on the web after it has been dried. Printing with an aqueous solution of the superabsorbent polyelectrolytes and crosslinker would be either in-line or off-line of a converting process to either side of the paper product. Nonwovens, absorbent paper products and textiles are suitably printed with super absorbent polyelectrolytes including crosslinker.

Rotogravure is an intaglio printing method offering precise superabsorbent polyelectrolyte application and transfer of a desired design image by use of precisely etched roller surface. Design total area can be varied by adjustment to small spaced engraved deposits (i.e., cells) in the roller surface. Design coverage can vary from 1% to 90% of coverage preferably 1-80% coverage. Engraving can be accomplished by chemical acid etch or electro-mechanical methods, with a preference for the latter method. The engraving will use a range between 100 to 200 lines per inch with engraving depths ranging between 5 to 50 microns.

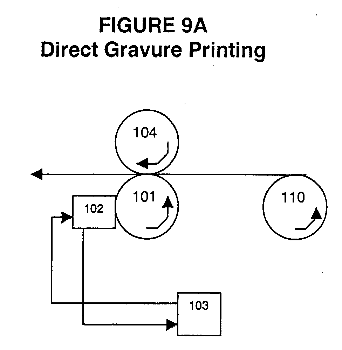

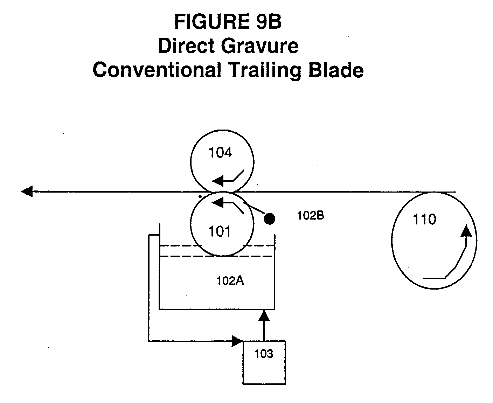

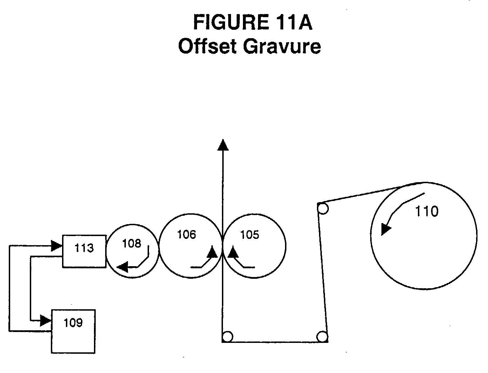

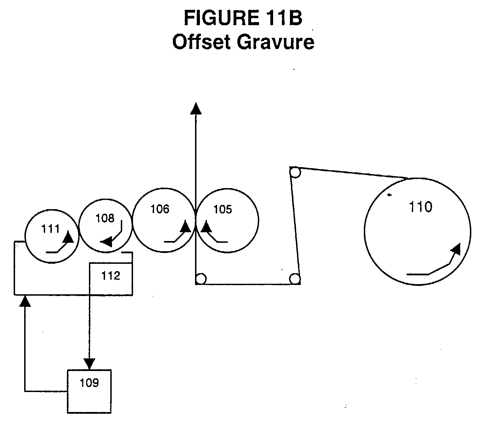

Direct rotogravure is the preferred gravure method of choice, as shown in Figures 9A and 9B, but offset gravure, illustrated in Figures 11A and 11B, are also suitable methods. The design image is transferred to the fibrous web or substrate when the web (Figure 9A, Number 110) is passed in contact between the engraved roller (101) and a covered impression roller (104). This impression roller (104) covering can be a natural or synthetic rubber with a durometer between 60 and 90 Shore A. Contact between the rollers will range from .250 to .625 inches. The aqueous superabsorbent polyelectrolyte including the crosslinker is recirculated from a supply source (103) to an applicator head (102), which is in contact with the engraved roller (101).

Either side of the nonwoven paper, textiles or absorbent paper can be printed using direct gravure as shown in Figures 9A and 9B. Both sides can be printed by use of two print stations in sequence. For absorbent paper products the printing can be conducted prior to embossing or after embossing.

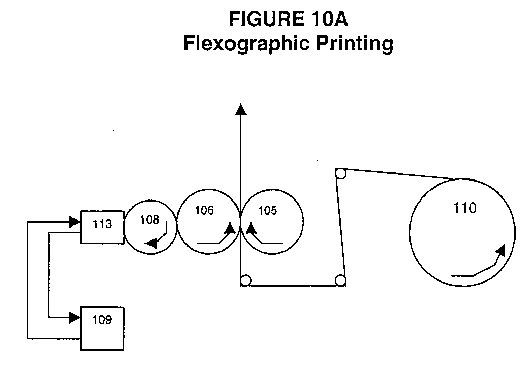

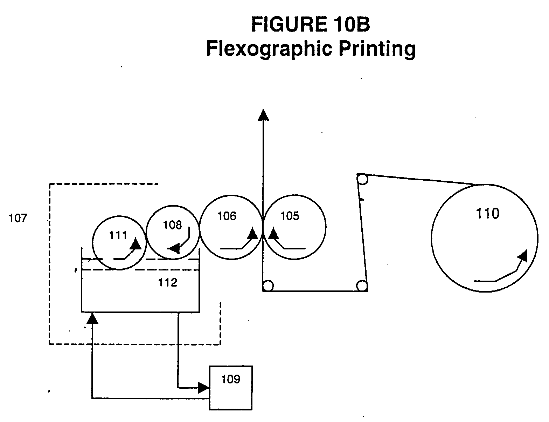

Flexographic printing, illustrated in Figures 10A and 10B, is a rotary relief printing method where the desired design image employing an elastometric material is raised above non-printing areas on a roller surface. The elastometric material can be molded or laser engraved natural or synthetic rubber, or photopolymer and is commonly referred to as a plate cylinder when mounted on a roller. A durometer range between 35 and 65 Shore A is used for the elastometric material.

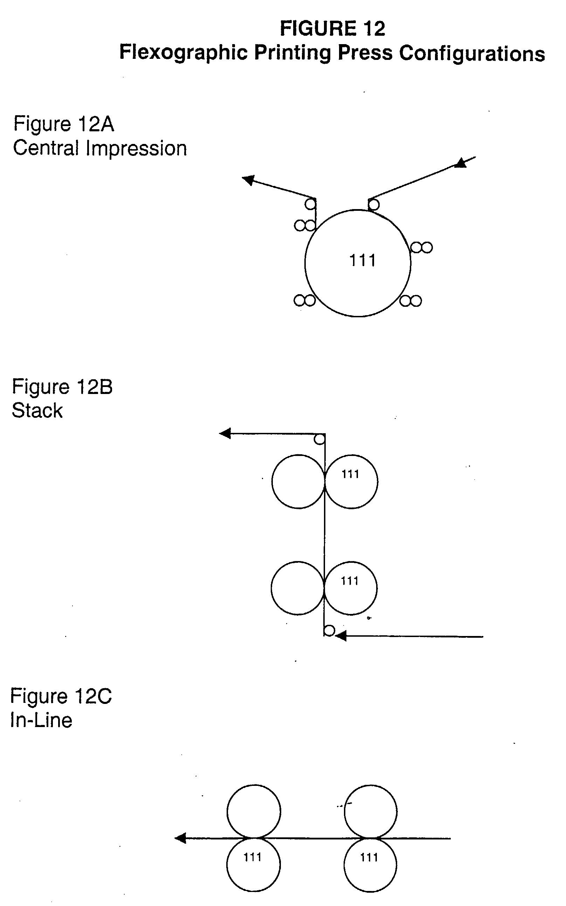

Aqueous superabsorbent polyelectrolyte including the crosslinker is transferred to the elastometric-raised image by means of an engraved roller referred to as an anilox roller. Engraved small spaced deposits can be varied to control the volume of the aqueous superabsorbent polyelectrolyte including the crosslinker transferred to the raised image when the anilox roller is in contact with the plate cylinder. The amount of this contact ranges between .002 to . 012 inch. The superabsorbent polyelectrolyte including the crosslinker is recirculated from a supply pump to an applicator head in direct contact with the engraved anilox roller. The engraved roller does not transfer the superabsorbent polyelectrolyte directly to the substrate, thus differs from the direct rotogravure method. The amount of polyelectrolyte including crosslinker transferred can be controlled by specification of the engraving volume. A range of volume between 1.0 and 10.0 billion cubic micron per square inch is suitable. The design image is transferred (Figure 10) from the plate to the paper when the web is passed in contact between the plate cylinder and an impression roller. This is shown in Figure 12A, 12B, and 12C or Figures 10A and 10B. The impression roller is commonly a metal roller or hard elastometric material. The amount of contact between the plate cylinder and impression roller ranges between .002 to .012 inch.

The textile, nonwovens, or paper products are printed with the aqueous superabsorbent polyelectrolytes including crosslinker utilizing a gravure or flexographic process. In the gravure process the printing image is engraved into a cylinder in the form of cells which become filled with the aqueous polyelectrolyte and crosslinker. Printing is achieved by passing the paper or textile between the gravure cylinder at Figure 9B (101) and an impression roller (104) under pressure.

The printing unit of a gravure press often consists of an aqueous superabsorbent electrolyte in fountain pan (102A) which the etched cylinder rotates in the aqueous superabsorbent electrolyte and crosslinker mixture. A metal or plastic doctor blade (102B), which reciprocates from side to side, scrapes excess superabsorbent electrolyte from the cylinder surface. The substrate is fed from reels into a nip between the etched cylinder and a rubber covered impression roller which supplies the pressure needed to transfer the aqueous superabsorbent electrolyte from the cells to the paper or textile substrate. The printed web may run through a heated drying system where the solvents are evaporated and extracted, and the superabsorbent polyelectrolyte is thus dried. The superabsorbent polyelectrolyte and crosslinker that is supplied to each unit, is pumped up to the polyelectrolyte fountain pan (102A) and continuously circulated, and usually viscosity control is incorporated in this system. Because each printing unit may have an integral drying system and impression roller, most presses consist of units arranged in line, as shown in Figure 12C, where the web travels between units in a horizontal plane. As the impression cylinder is not gear driven, but obtains its drive through contact with the gravure cylinder, cylinders of different size can be used to provide variable print repeat dimensions within certain limits.

The function of the doctor blade is to remove surplus superabsorbent polyelectrolyte from the surface of the cylinder leaving the polyelectrolyte in the cells. There are many possible configurations for the doctor blade and they have an effect on the printed result. The thickness of the blade is generally 0.006 to 0.040 inches. Doctor blades in reciprocating designs are usually supported by a backing blade to give extra support. A reverse angle manifold system can be utilized (Figure 9A) where the doctor blade does not normally require oscillation.

Doctor blades are normally made to reciprocate by up to 6 cm. This gives a better wipe and disperses paper fibers that may get trapped under the blade. Blade mountings must have adjustments to cope with different sizes of cylinder and also movement for making the blade exactly parallel with the cylinder axis.

The impression roll has a steel core with a rubber covering. It is a relatively hard rubber up to 90 shore A durometer and the pressure applied between it and the printing cylinder is high in relation to other processes.

Gravure configurations are set forth in Figures 9A and 9B. Most gravure printing is done on web-fed presses, which provide facilities for supporting and controlling the supply reel during unwinding. A variety of equipment can be used for both manual and automatic splicing. Tension control systems are used to provide stability of web movement to the first printing unit and through multiple units including the last print unit. Most often, gravure presses are of an in-line design as shown in Figure 12C.

Flexography is a rotary print process in which the printing images are raised above non-printing areas like that in the letterpress process. Figures 10A and 10B illustrate preferred flexographic processes utilized in the printing of the aqueous superabsorbent polyelectrolytes on the paper or textile surface.

A low printing pressure is used in the process because of the relatively soft printing plates that are suitably used.

In the flexographic process, the application of aqueous superabsorbent polyelectrolyte and crosslinker to the surface of the printing plate is conducted by means of an engraved (anilox) roller. The result is a simple polyelectrolyte feed system that consists of not more than two rollers (Figure 10B) for a conventional design.

Although most flexographic printing is reel to reel, the machines enable relative changes in the print repeat length to be made simply based on the press gearing.

The printing unit consists of three basic parts as shown in Figure 10A, 10B, and 10C:

(1) the aqueous superabsorbent polyelectrolytes unit (107);
(2) the plate cylinder (106); and
(3) the impression cylinder (105).

The function of the aqueous superabsorbent polyelectrolyte system is to meter out a fine and controlled film of liquid superabsorbent polyelectrolyte, and apply this to the surface of the printing plate (106). The polyelectrolyte application system consists basically of a polyelectrolyte fountain pan (112), a rubber covered fountain roller (111), and an engraved (Anilox) (108) polyelectrolyte roller into which cells of uniform size and depth are engraved. The fountain roller lifts polyelectrolyte to the nip position, where it is squeezed into the cells in the screened polyelectrolyte roller and by a shearing action is removed from the roller surface. The polyelectrolyte in the cells is then transferred to the surface of the printing plates. To regulate polyelectrolyte film thickness in printing, engraved polyelectrolyte rollers are suitably utilized which have volumes of from 1.0 to 10.0 billion microns per square inch (bcm/in$^2$) or greater. These may be engraved or etched metal or ceramic. The engraved cells are generally square in shape with sloping sidewalls. The number of cells and their configuration regulate the volume of polyelectrolyte transferred. Further regulation of the polyelectrolyte is achieved by varying the surface speed of the fountain roller (111), altering the pressure between the fountain roller (111) and engraved roller, and also altering the hardness of the rubber covering on the fountain roller. A reverse angle manifold system can be utilized (Figure 10A) which replaces the fountain pan and rubber roller in a conventional system.

The plate cylinder is usually made from steel. The printing plates, which can vary in thickness between 0.042-.250 inches or greater, are most often secured to the cylinder with two-sided, self-adhesive material.

The impression cylinder is most often made from steel. The substrate passes between the plate and impression cylinders, which generate printing pressure. The polyelectrolyte and the crosslinker are transferred from the cells in the screened polyelectrolyte roller to the plate surface, and then to the substrate, during which both reach virtually a uniform film.

In our process, a central impression (Figure 12A) configuration of flexographic press can be utilized. Also the stack and in-line press can be used (see Figure 12B and 12C). The stack press (Figure 12B) consists usually of two or more integral printing units arranged in vertical formation. This machine enables reverse side printing on the web.

The common impression machine (Figure 12A) consists of a large cylinder around which are arranged either four or more printing units. The cylinder is very accurately made from steel. Usually the web enters the top or bottom unit on one side of the cylinder, travels to each unit with the cylinder, and emerges from the top or bottom unit on the opposite side of the cylinder.

The in-line machine (Figure 12C) which is a less common configuration for wide web applications, consists of printing units arranged in horizontal formation, with the impression cylinder situated below the web, thus providing easy access to the plate cylinder. The web passes through each printing unit in a horizontal path.

Many products printed by flexography are required in reel form for subsequent processing, and so machines provide suitably versatile winding equipment.

The machine also provides facilities for supporting and controlling the supply reel during unwinding. A variety of equipment is available for both manual and automatic splicing and also tension control.

A polyelectrolyte drying system can be provided as part of the press design.

There are several kinds of image carrier in flexography, each of which is suitable for use in our process:

(1) the traditional molded rubber plate;
(2) the photopolymer plates; and
(3) the laser engraved rubber plates or rubber rollers.

There are various photopolymer plate material suitable for flexographic printing. These plates are made directly from photographic negatives.

## EXAMPLE 1

A superabsorbent polymer for H.B. Fuller, Fulatex PD-8081-H, the sodium salt of polyacrylate having a molecular weight of about 190,000 to 210,000, was applied to unbonded air laid nonwoven web, consisting of wood pulp fibers only.

The Fulatex binder was mixed with an additive, Bacote 20 (ammonium zirconium carbonate solution), and then diluted to a 10% solution. While the Fulatex was slowly agitating, the Bacote 20 was very slowly added until uniform (approximately 5 minutes). The water was added to decrease the viscosity of the solution for improved spraying. Another solution was made the same way with the addition of a surfactant. A control (non-superabsorbent) binder was also made for a comparison. The binder formulas are shown below.

| Formula A | |
|---|---|
| Fulatex PD-8081-H (23%) | 380.8 |
| Bacote 20 (42.5%) | 19.2 |
| Water | 557.4 |
| | 957.4 |
| | (10.0% solids) |

| Formula B | |
|---|---|
| Fulatex PD-8081-H (23%) | 380.8 |
| Bacote 20 (42.5%) | 19.2 |
| Water | mix 557.4 |
| Aerosol OT-75 | 1.0 |
| | (10.0% solids) |

| Formula C | |
|---|---|
| Airflex 135 | 384.6 |
| Water | 611.9 |

(continued)

| Formula C | |
|---|---|
| Sodium Bisulfate | 2.0 |
| Aerosol OT-75 | 1.5 |
| | (20% solids) |

The binder formulas were applied to unbonded air laid handsheets by spray bonding. Formulas B and C used a conventional dioctyl sodium sulfosuccinate surfactant as an additional control to verify wetting rates of my invention when compared to conventional binder systems. The handsheets were oven dried at about 80°C.

The handsheets were tested for physical properties. The averages of the individual tests are shown in the table below.

| | A | B | C |
|---|---|---|---|
| | Fulatex PD-8081-H | Fulatex PD-8081-H with surfactant | Control |
| Basis Weight (Ib/ream) | 60.5 | 61.1 | 61.1 |
| % Binder in Sheet | 21.8 | 21.9 | 22.0 |
| 1 Ply Bulk (mils) | 36 | 38 | 35 |
| Dry Tensile (g/in.) | 1595 | 1655 | 1810 |
| Wet Tensile (g/in.) | 45 | 40 | 985 |
| Absorbency Rate (sec.) | 1.6 | 2.2 | 4.2 |
| Absorbent Capacity | 22.0* | 21.3* | 9.7* |
| (XOW) | 39.0** | 35.0** | |

*30 sec. soak

**Minute soak

The Fulatex PD-8081-H gives good dry tensile strength, an improved absorbency rate and a very high absorbency capacity.

Table 1 shows clearly that my invention uses a polyelectrolyte that serves both as a binder and as a superabsorbent. In this experiment, a final basis weight of 52 grams per square meter (gsm) was chosen.

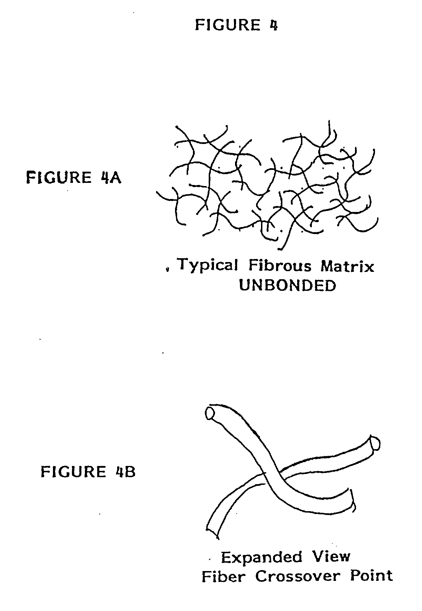

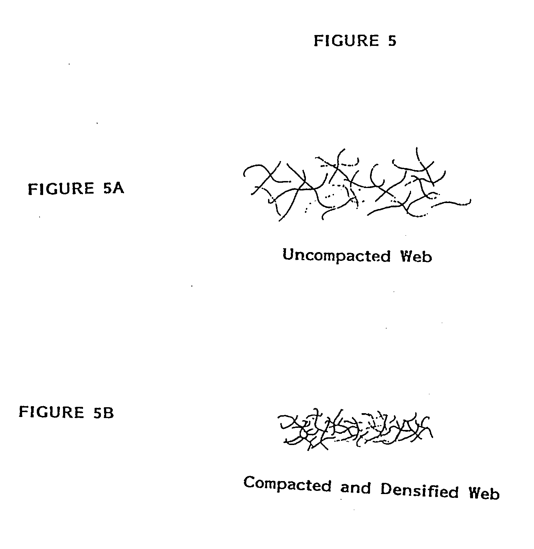

In Cell 1, for wood pulp only without any mechanical compression no tensile strengths could be measured, clearly showing that a wood pulp furnish alone has very limited usefulness. Figures 4A and 4B show a simple matrix of fibers in an unbonded and uncompressed stage, and also a typical fiber crossover point. Compaction or densification of this web was achieved by flat pressing the web. This is designed to squash the fibers together, and increase mechanical entanglement and/or hydrogen bonding. It also changes the pore size between the fibers, making it smaller. This action led to tensiles of 90gr/in., which is still far too low to be useful. Figures 5A and 5B show the uncompacted and compacted fibrous webs.

In Cell 2, 20% of the wood pulp has been replaced with Superabsorbent Fibers (SAF), which is a typical loading amount used in such product constructions. This is generically represented by Figure 1B in that distinction is made for the two different types of fiber (SAF and wood pulp). Again, in the uncompacted stage, no tensiles could be measured. But as a function of compaction the tensiles increased to 190 gr/in. It is thought that the increase in compacted tensiles is largely due to increased hydrogen bonding, but the exact mechanism is not known. The increased tensiles are still low enough so as to severely limit any handling of the web thus impairing its usefulness.

In Cell 3, the SAF has been replaced by the Superabsorbent Binder (SAB) of my invention. This is represented by Figure 2C which shows fibers bonded at a crossover point with a chemical binder which for Cell 3 is the superabsorbent binder of my invention. Now the tensile strengths without compaction have been raised to 2200 gr/in., clearly indicating that the polyelectrolyte is acting as a binder and is developing significant strength in the web. Now that the web has integrity, we were also able to measure its water holding capacity (WHC) and found that this web exhibited 19 times its own weight in water pickup (XOW).

By contrast, Cell 6 bonded a web with a conventional ethylene vinyl acetate binder that does not exhibit superabsorbent properties. This, too, is represented by Figure 2C but for Cell 3 the chemical binder would be a conventional chemical binder. The tensile strengths were similar (2000 gr/in.), but the WHC was only 7 XOW, again, clearly indicating the dual benefits of my invention.

In Cell 4, combinations of SAF and SAB were used to check for the effects on tensile and WHC. This is shown by Figure 1B in the unbonded state and Figure 2C in the bonded state. Tensiles dropped to 1700 gr/in, which appears to be an indication that SAF bonds are not as strong as wood pulp bonds in a chemically bonded web. Furthermore, although the WHC increased to 33 XOW, the increase was not linear in proportion to the extra amount of superabsorbent material added.

And in Cell 5, SAF, SAB, and Superabsorbent Powder (SAP) were added to the web. This is best shown by Figure 2D, which, for this example would utilize at least one of the fibers as an SAF fiber and clearly shows the positioning of the SAP and SAB. In Cell 5 the tensiles decreased even further to 625 gr/in. This was somewhat expected as the powders contribute no bonding strength to the final web. And, while the WHC increased only slightly to 37 XOW, it was not linear in proportion to the amount of total absorbent in the web.

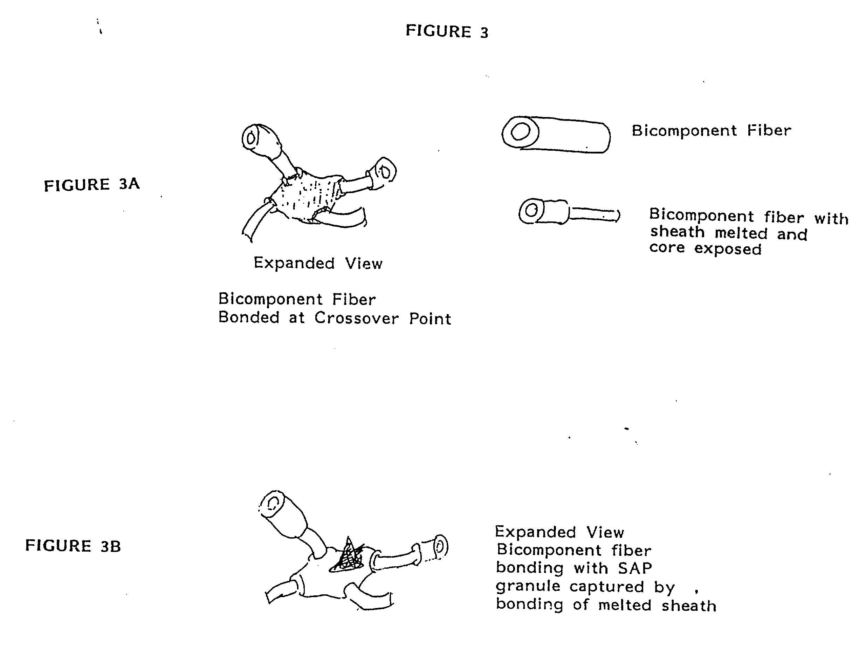

Figure 3A shows the nature of a web bonded from bicomponent fibers. Wood pulp and/or SAF and other synthetic fibers could easily be added to such webs, but they would still suffer from the comparative lack of tensiles, and from heavy linting. SAP could also be added to such webs (as is shown in Figure 3B), but they would suffer from the same deficiencies of inadequate bonding between the bicomponent fibers and the SAP, causing poor retention of the SAP. A good fix for the deficiencies of thermal bonded bicomponent webs is to add a surface coating of binder to tie down the loose fibers and/or SAP. In my invention, SAB would preferably be used as the tie down binder.

Table 1

| Physical Properties | | | |
|---|---|---|---|
| Cell 1 | 52 grams wood pulp | 0 tensile unpressed 90 gr/in. pressed | mechanical bonding hydrogen bonding |
| Cell 2 | 41.6 gsm wood pulp 10.4 grams SAF | 0 tensile unpressed 190 gr/in pressed | mechanical bonding increased hydrogen bonding from SAF |
| Cell 3 | 41.6 gsm wood pulp 10.4 gsm SAB | 2200 gr/in tensile | SAB acting as binder 19 x 0W holding capacity |
| Cell 4 | 10.4 gsm SAF 10.4 gsm SAB 31.2 gsm wood pulp | 1700 gr/in tensile | SAF fibers decrease tensile strength compared to wood pulp fibers when bonded into SAB 33 x OW holding capacity not linear increase with SAF |
| Cell 5 | 20.8 gsm wood pulp 10.4 gsm SAF 10.4 gsm SAP 10.4 gsm SAB | 625 gr/in tensile | Tensile decreases further with SAP addition at instance basis wt. 37 x OW holding capacity not linear increase with SAP |
| Cell 6 | 41.6 gsm wood pulp 10.4 gsm conventional EVA binder (non-SAB) | 2000 gr/in | 7 x OW |

Other embodiments of the invention will be apparent to those skilled in the art from consideration of the specification and practice of the invention disclosed herein. It is intended that the specification and examples be considered as exemplary only with the true scope and spirit of the invention being indicated by the following claims.

## Claims

1. A superabsorbent fibrous web of wood pulp or cellulosic fibers, natural fibers, synthetic fibers, or any mixture thereof preparable by treating the web with an aqueous solution of a superabsorbent polyelectrolyte.

2. A superabsorbent fibrous web according to Claim 1 which comprises a non-woven web and wherein the crosslinked superabsorbent polyelectrolyte replaces conventional binders.

3. A superabsorbent fibrous web according to Claim 1 or Claim 2 wherein the polyelectrolyte is a copolymer of:

   an olefinically unsaturated acid;
   an olefinically unsaturated acid ester; and, optionally,
   an olefinically unsaturated acid hydroxy ester;

or a salt thereof.

4. A superabsorbent fibrous web according to Claim 3 wherein the acid, acid ester or hydroxyester is a carboxylic acid, carboxylic acid ester or carboxylic acid hydroxyester, respectively; or a salt thereof.

5. A superabsorbent fibrous web according to Claim 3 or 4 wherein the acid is acrylic acid, methacrylic acid, itaconic acid, mesaconic acid, citroconic acid, aconitic acid, or a mixture thereof; or a salt thereof.

6. A superabsorbent fibrous web according to Claim 3, 4 or 5 wherein the ester is ($C_1$ to $C_{10}$ alkyl) acrylate or a ($C_4$ to $C_{10}$ alkyl) methacrylate, or a mixture thereof.

7. A superabsorbent fibrous web according to any one of Claims 3 to 6 wherein the hydroxyester is an ($\Omega$-hydroxy $C_1$ to $C_4$ alkyl) acrylate, or a mixture thereof.

8. A superabsorbent fibrous web according to Claim 6 or 7 wherein the ester or hydroxyester is selected from methyl acrylate, ethyl acrylate, propyl acrylate, hexyl acrylate, butyl methacrylate, hexyl methacrylate, octyl methacrylate, decyl methacrylate, 2-hydroxyethyl acrylate, hydroxymethyl acrylate, 3-hydroxyl-propyl acrylate, 4-hydroxybutyl acrylate and mixtures thereof.

9. A superabsorbent fibrous web according to any one of Claims 3 to 8 wherein the copolymer comprises:

> from 50 to 92 percent by weight of olefinically unsaturated acid;
> from 8 to 50 percent by weight of olefinically unsaturated acid ester; and
> from 0 to 15 percent by weight of olefinically unsaturated acid hydroxyester.

10. A superabsorbent fibrous web according to any one of Claims 3 to 9 wherein the copolymer is preparable from acrylate comonomers.

11. A superabsorbent fibrous web according to any preceding claim wherein the polyelectrolyte is a sodium, lithium or potassium salt.

12. A superabsorbent fibrous web according to any preceding claim wherein the polyacrylate has a molecular weight in excess of 150,000, for example from 150,000 to 220,000.

13. A superabsorbent fibrous web according to any preceding claim wherein the web is treated with a crosslinking agent.

14. A superabsorbent fibrous web according to Claim 12 wherein the crosslinking agent comprises a multivalent cationic species.

15. A superabsorbent fibrous web according to Claim 13 or 14 wherein the crosslinking agent comprises a zirconium compound having a valence of plus four.

16. A superabsorbent fibrous web according to Claim 15 wherein zirconium compound is selected from the group consisting of ammonium zirconium carbonate, zirconium acetylacetonate, zirconium acetate, zirconium carbonates, zirconium sulfate, zirconium phosphate, potassium zirconium carbonate, zirconium sodium phosphate, sodium zirconium tartrate, and mixtures of these.

17. A superabsorbent fibrous web according to any one of Claims 13 to 16 wherein the crosslinker is selected from the following 1,3-dichloroisopropanol, 1,3-dibromoisopropanol; tetrahydrothiophene adduct of novolac resins; epichlorohydrin, epibromohydrin, 2-methyl epichlorohydrin and glycerine diglycidyl ether, ethylene glycol diglycidyl ether, propylene glycol diglycidyl ether, diethylene glycol diglycidyl ether; and the mixtures of these.

18. A superabsorbent fibrous web according to any one of Claims 13 to 17 wherein the web is treated and bonded with the aqueous solutions of superabsorbent polyelectrolytes and crosslinkers.

19. A superabsorbent fibrous web according to any one of Claims 13 to 18 wherein the polyelectrolytes and crosslinkers are applied as a topcoat of the web to enhance superabsorbent properties.

**20.** A superabsorbent fibrous web according to any of Claims 13 to 19 wherein the polyelectrolytes and crosslinkers are applied as a laminate adhesive to hold two or more webs together.

**21.** A superabsorbent fibrous web according to any preceding claim wherein the synthetic fiber is selected from the group consisting of regenerated cellulose polyolefins, polyethylenes, polypropylenes, polyesters, polyimides, polyamides, inorganic polymers, silica polymer, and carbon fibers.

**22.** A superabsorbent fibrous web according to any preceding claim wherein the web is an air laid non-woven web; or is produced by a carding process; or is produced by a melt blown process; or is produced by a spunbond process; or is produced by a coforming process; or is in the form of a textile web; for example formed by a weaving process, or by a knitting process.

**23.** A superabsorbent fibrous web according to any preceding claim wherein the web is treated by spraying; by printing; by padding; by saturating; or by coating one or both sides with the aqueous solution of the superabsorbent polyelectrolyte, and where present, crosslinking agent.

**24.** A superabsorbent fibrous web according to any preceding claim formed as a towel; an absorbent paper product; feminine hygiene pad; incontinence pad; adult diaper; baby diaper; wound dressing; meat soaker pad; wipers; cable wrap paper; agricultural moisture retention system; or medical drape.

# FIGURE 1

### FIGURE 1A

Typical Fibrous Matrix

UNBONDED

### FIGURE 1B

Expanded View

Superabsorbent Fiber

Other Fiber

Expanded View
Fiber Crossover Point

EP 0 992 250 A2

FIGURE 2

SAP Powder

EP 0 992 250 A2

FIGURE 2A

Superabsorbent granules (powders) are smaller than some of the web interstial holes.

Unbonded, uncompacted fibrous matrix with superabsorbent powder.

SAP powder

FIGURE 2B

More SAP is trapped in smaller interstitial holes

Unbonded compacted (densified) fibrous material with super absorbent powder

FIGURE 2 – Continued

FIGURE 2C

Chemical Binder

Two fibers bonded with chemical

Expanded View

SAP powder

Chemical Binder

FIGURE 2D

Two fibers bonded with chemical and capturing a SAP particle

EP 0 992 250 A2

FIGURE 3

FIGURE 3A

Bicomponent Fiber

Bicomponent fiber with
sheath melted and
core exposed

Expanded View

Bicomponent Fiber
Bonded at Crossover Point

EP 0 992 250 A2

FIGURE 3B

Expanded View
Bicomponent fiber
bonding with SAP
granule captured by  ,
bonding of melted sheath

FIGURE 4

FIGURE 4A

Typical Fibrous Matrix
UNBONDED

FIGURE 4B

Expanded View
Fiber Crossover Point

EP 0 992 250 A2

# FIGURE 5

EP 0 992 250 A2

## FIGURE 5A

Uncompacted Web

## FIGURE 5B

Compacted and Densified Web

FIGURE 6

FIGURE 7

FIGURE 8

THROUGH-AIR-DRYING (TAD) PROCESS

# FIGURE 9A
## Direct Gravure Printing

# FIGURE 9B
## Direct Gravure
## Conventional Trailing Blade

104

102B

101

110

102A

103

# FIGURE 10A
## Flexographic Printing

## FIGURE 10B
## Flexographic Printing

## FIGURE 11A
## Offset Gravure

# FIGURE 11B
## Offset Gravure

## FIGURE 12
## Flexographic Printing Press Configurations

Figure 12A
Central Impression

Figure 12B
Stack

Figure 12C
In-Line

# FIGURE 13

## Typical Card & Bond Line

| | |
|---|---|
| 120 BALE OPENER | 127 DOFFER ROLL |
| 121 FINE OPENER | 128 PEELER ROLL |
| 122 CHUTE FEED | 129 UNBONDED CARDED WEB |
| 123 FEED ROLL | 130 LATEX APPLICATION SYSTEM |
| 124 LICKERIN ROLL | 131 DRYER |
| 125 WORKER/STRIPPER ROLLS | 132 BONDED CARDED WEB |
| 126 MAIN CYLINDER | 133 TAKE UP REEL |